# EUROPEAN PATENT SPECIFICATION

(11) **EP 0 983 027 B1**
(45) Date of publication and mention of the grant of the patent: **28.12.2005**
(21) Application number: 98921235.2
(22) Date of filing: 18.05.1998
(51) Int. Cl.: A61B 17/12, A61M 25/10

(54) **DEVICE FOR PARTIALLY OCCLUDING BLOOD VESSELS**
VORRICHTUNG ZUR TEILWEISEN OKKLUSION VON BLUTGEFÄSSEN
DISPOSITIF DESTINE A OBSTRUER PARTIELLEMENT DES VAISSEAUX SANGUINS

(30) Priority: 19.05.1997 US 46977 P
(43) Date of publication of application: 08.03.2000
(73) Proprietor: Cardio Medical Solutions, Inc., Fountain Valley, CA 92708 (US)
(72) Inventor: NOBLES, Anthony, A., Huntington Beach, CA 92648 (US)
(74) Representative: De Clercq, Ann G. Y.
(86) International application number: PCT/US1998/010018
(87) International publication number: WO 1998/052476

(56) References cited:
- EP-A- 0 839 550
- WO-A-97/12540
- US-A- 3 903 893
- US-A- 4 230 119
- US-A- 4 307 722
- US-A- 5 312 344

## Description

### BACKGROUND OF THE INVENTION

### 1. Field of the Invention

This invention relates to a device and method for creating a region of hemostasis along a wall of a blood vessel without interrupting the flow of blood through the blood vessel. The device may be used, for example, for creating a working area along the aorta for performing a cardiac bypass procedure.

### 2. Background Discussion

Coronary artery diseases are often caused by atherosclerosis or narrowing of the small arteries between the aorta and the heart muscles. There are several ways to provide blood flow around occluded segments of arteries or veins, however, the known methods cause a large amount of trauma to the patient. One method is to perform an "open heart surgery," which is cracking open the chest and exposing the heart and treating the vessel directly. However, the large incision and surgically cut sternum take a long time to heal.

Another method is to perform a bypass operation wherein a section of the saphenous vein, or a suitable substitute, is grafted, usually between the ascending aorta just above the heart and one or more of the coronary arteries beyond the points of blockage. The bypass operation is performed with the patient connected to a heart-lung machine and the heart is stopped. Because the heart is stopped, the heart-lung bypass can damage blood cells. Additionally, the patient's internal body temperature is reduced while on a heart-lung bypass to reduce basil metabolism and then the body temperature is increased to normal when the procedure is over. This thermal change to a person's body can cause damage to the intestinal track as well as causing additional stress to the patient.

If the patient is not placed on a heart-lung bypass, the aorta is typically partially clamped along its axis to create an area of blood stasis and a small channel for blood flow. However, clamping the aorta can cause injury to the aorta and can also cause plaque formations to break off into the blood stream and cause vascular accidents such as strokes and emboli.

A micro-hemostat was developed (US 4,230,119) including a double walled tubular bar including cuff portions of highly elastic material adjacent each end of the bar. In use, the bar is pressurized to inflate the cuff portions to form seals with the inner wall of the blood vessel while the blood can flow through the vessel only by passing through the lumen of the bar.

The cuff portions, however, inflate like a sphere and contact the vessel over a large contact area causing again a severe risk for plaque formations to break off into the blood.

What is needed therefore is a device and method for performing a cardiac bypass procedure without the need to clamp the aorta or place the patient on a heart-lung bypass machine.

### SUMMARY OF THE INVENTION

The present invention involves a device for partially occluding the blood vessel such that an area of hemostasis within the blood vessel is created without interrupting the blood flow through the blood vessel. In one embodiment, the device comprises a flow-through balloon that is adapted to be introduced into the aorta through an incision. The balloon includes a tubular portion having first and second partially occlusive portions which extend radially therefrom. When the balloon is inflated, a region of hemostasis is created along an outside wall of tubular portion while blood continues to flow through the tubular portion. The device thus creates an anastomosis site along a wall of the aorta without the need to stop the patient's heart, use a cross clamp, or connect the patient to a heart-lung machine.

In accordance with one aspect of the invention, the device comprises a first and second partially occlusive portions that are spaced apart from one another, wherein the partially occlusive portions are inflatable to at least the inner diameter of the blood vessel, and a tubular member that interconnects the first and second partially occlusive portions and forms a conduit which allows the blood to flow through, wherein the diameter of the tubular member is smaller than the diameter of the first and second partially occlusive portions. Furthermore, the inflation of the first and second partially occlusive portions creates an area of hemostasis between an outer surface of the tubular member and an inner wall of the blood vessel while blood continues to flow through the tubular portion.

In accordance with an additional aspect of the invention, it is preferable that the first and second partially occlusive portions and the tubular member are made of a single, continuous one-piece inflatable unit, such that the first and second partially occlusive portions and the tubular member are inflatable. It is further preferable that the inflatable first and second partially occlusive portions and tubular member are made from a low compliance biocompatible material. In addition, the partially occlusive portions are sized to create a region of hemostasis within a human aorta.

In accordance with an additional aspect of the invention, the device preferably has a tube that is coupled to the first and second portions and adapted to be inserted directly into an incision in the blood vessel to deploy the partially occlusive portions within the blood vessel. The tube can further contain at least one inflation lumen formed therein, the inflation lumen fluidly coupled to the tubular member and the first and second partially occlusive portions. The tube can further contain a lumen having a suture loop therein, the suture loop coupled to at least one of the inflatable partially occlusive portions such that the application of a force to the loop from outside the blood vessel urges the at least one partially occlusive portion to a collapsed state.

### BRIEF DESCRIPTION OF THE DRAWINGS

These and other features of the invention will now be described with reference to the drawings of a preferred embodiment, which is intended to illustrate and not to limit the invention, and in which:
Figure 1 is a perspective view of a device for partially occluding blood vessels using flow-through balloon in accordance with the invention.
Figure 2 generally illustrates the use of the device as used in a blood vessel, with the device shown in a deflated state.
Figure 3 generally illustrates the use of the device as used in a blood vessel, with the device shown in an inflated state.
Figure 4 is a perspective view of the device, illustrating a suture threaded through a proximal end of the balloon to facilitate collapsing the balloon for removal.
Figures 5A, 5B and 5C are side views in partial cross section which illustrate the use of a suture loop to cause the balloon to collape for removal.
Figure 6 is a side view of a mandrel that may be used to form a single, continuous one-piece balloon member, with a balloon member shown thereon in cross-section.
Figure 7 is a cross-sectional side view of a single, continuous one-piece member formed using the mandrel of Figure 6, with the enclosed end trimmed to create an opening.
Figure 8 is a cut away view of the device, illustrating how the balloon member of Figure 6 is folded into itself to create the device in accordance with the invention.

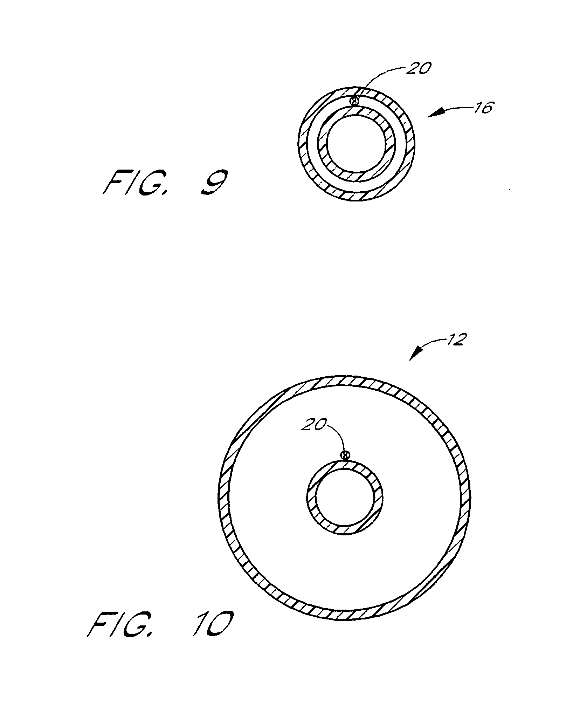

Figure 9 is a cross-sectional view taken along the line 9-9 of Figure 8.
Figure 10 is a cross-sectional view taken along the line 10-10 of Figure 8.
Figure 11 illustrates the use of the device in a human aorta.
Figure 12 illustrates the use of the device to treat an aneurysm in a blood vessel.

### DETAILED DESCRIPTION OF PREFERRED EMBODIMENT

In accordance with one embodiment of the present invention, a device for partially occluding blood vessels using flow-through balloon is described herein. In order to fully specify this preferred design, various embodiment specific details are set forth. It should be understood, however, that these details are provided only to illustrate this single preferred embodiment, and are not intended to limit the scope of the present invention.

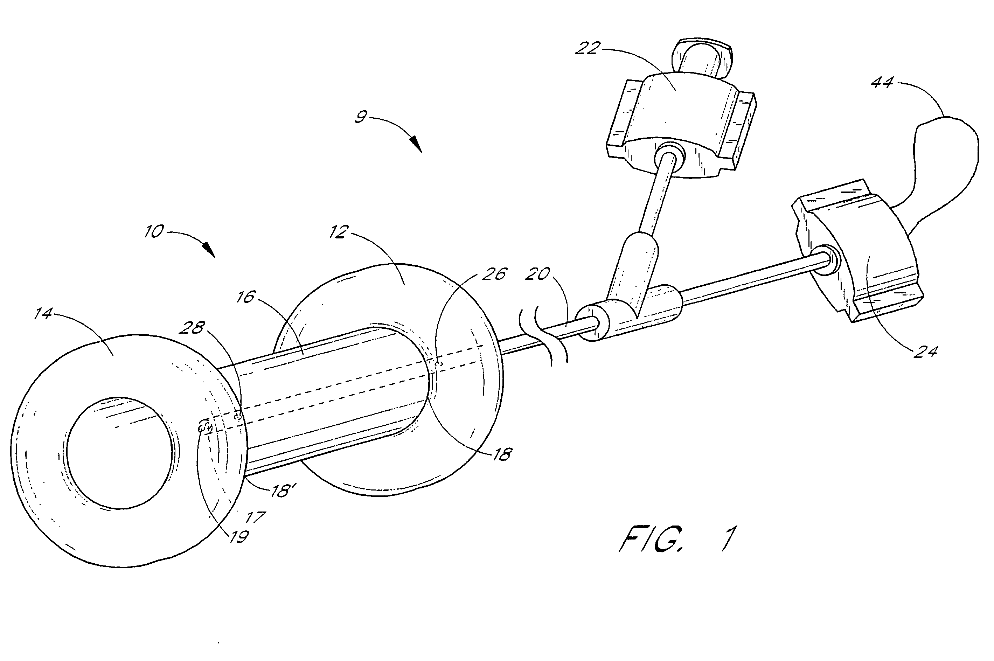

With reference to FIG. 1, the present invention provides a device 9 for partially occluding blood vessels using flow-through balloon 10. The balloon 10 comprises a proximal portion 12 and a distal portion 14 interconnected by a tubular member 16. In the illustrated embodiment, the proximal portion 12, the distal portion 14 and the tubular member 16 are made of a single, continuous one-piece balloon member that provides a single, inflatable chamber. However, it will be recognized that the tubular member 16 can be a separate unit, which interconnects the proximal portion 12 and the distal portion 14. In addition, the balloon 10 can alternatively be provided with two or more chambers that are separately inflatable.

As further illustrated in FIG. 1, a tube 20 with multiple lumens is coupled to the balloon 10. In the illustrated embodiment in FIG. 1, two lumens are contemplated. The first lumen is an inflation lumen 17 used to inflate the proximal portion 12 through an opening 26 and to inflate the distal portion 14 through an opening 28. The openings 26 and 28 are placed in the inflation lumen 17 and are longitudinally aligned with the proximal portion 12 and the distal portion 14, respectively. Access to the inflation lumen 17 is provided by a standard lure connector 22, which is adapted to receive a syringe (not shown). It will be recognized that the tube 20 can accommodate an additional inflation lumen (not shown), for example, such that each of the proximal portion 12 and the distal portion 14 can be inflated independently of each other. Using the syringe, the balloon 10 (including the proximal and distal portions and tubular member 12, 14, 16) can be inflated with an appropriate fluid such as air or saline.

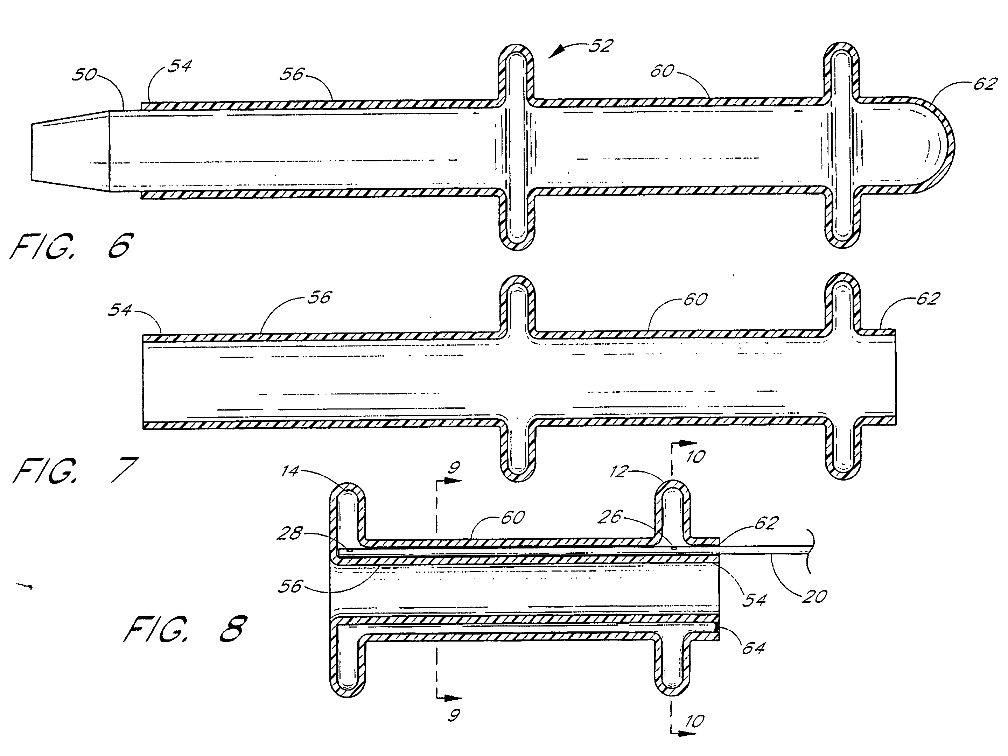

Alternatively, the balloon 10 can be constructed such that the proximal portion 12 and the distal portion 14 can be inflated without inflating the tubular member 16. Specifically, as illustrated in FIG. 1, a first seal 18 can be made around the junction between the proximal portion 12 and the tubular member 16, and a second seal 18' can be made at the junction between the distal portion 14 and the tubular member 16. The seals 18, 18' are formed between the inner and outer layers (Figure 8) of the balloon 10, and prevent fluid from entering the tubular member 16.

The second lumen 19 in the illustrated embodiment of FIG. 1 is a suture lumen used to collapse the balloon 10 from the proximal portion 12. The suture lumen is coupled to a second lure connector 24. The suture lumen will be discussed in more detail below.

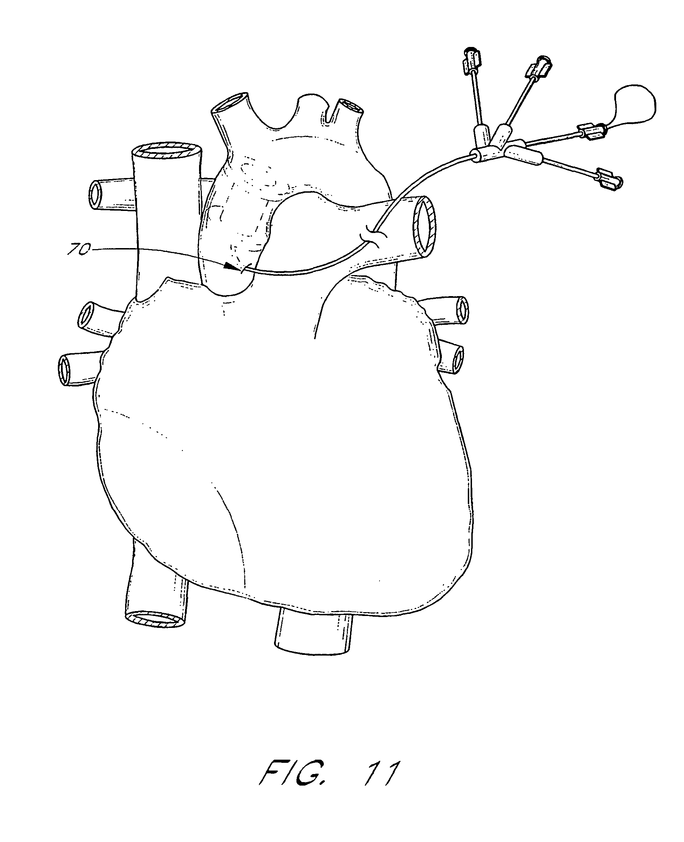

In other embodiments, additional lumens and lure connectors may be provided for performing additional functions. For example, in the embodiment depicted in Figure 11, a third lumen-connector pair is provided to administer cardioplegia or other medications, and a fourth pair is provided to monitor blood pressure.

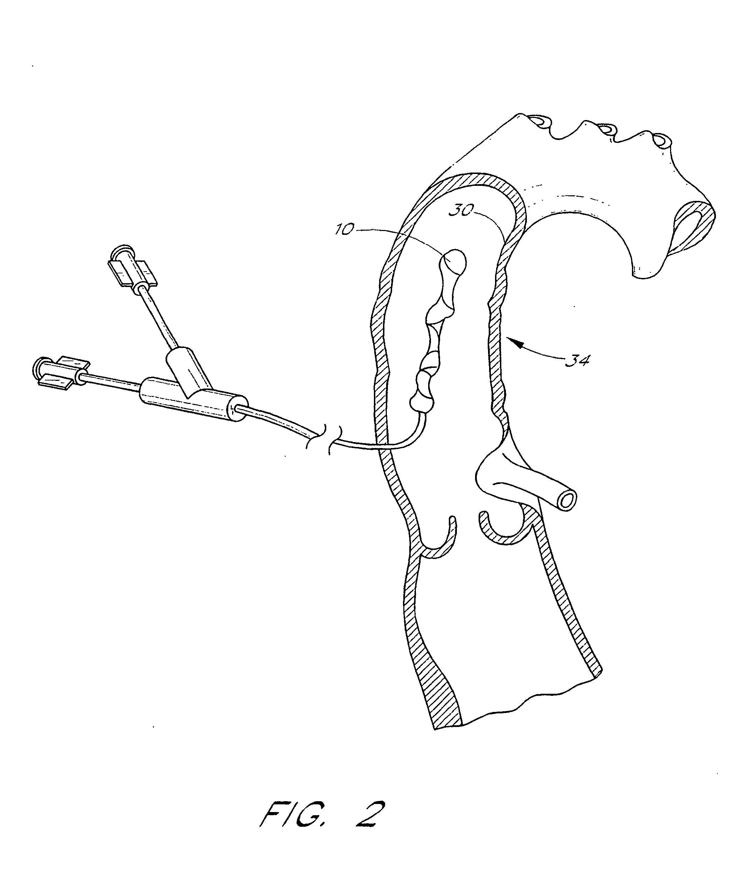

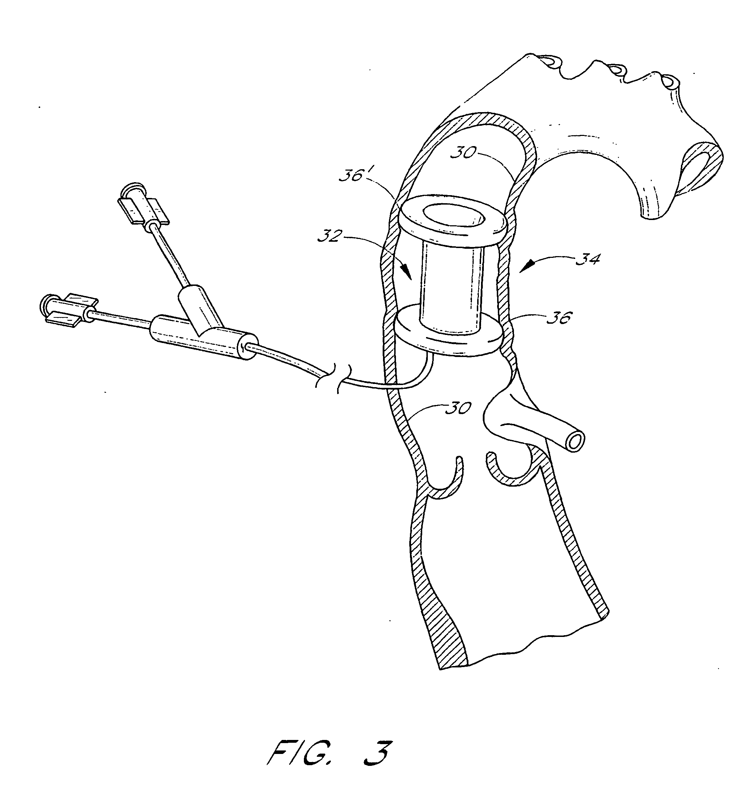

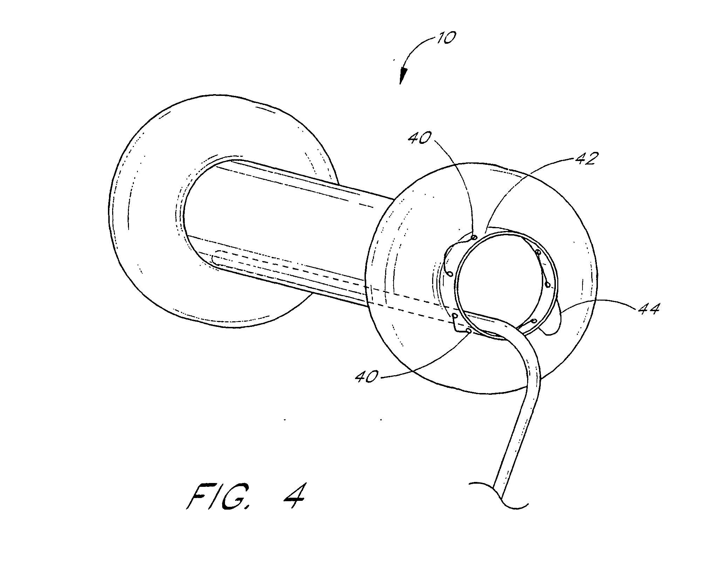

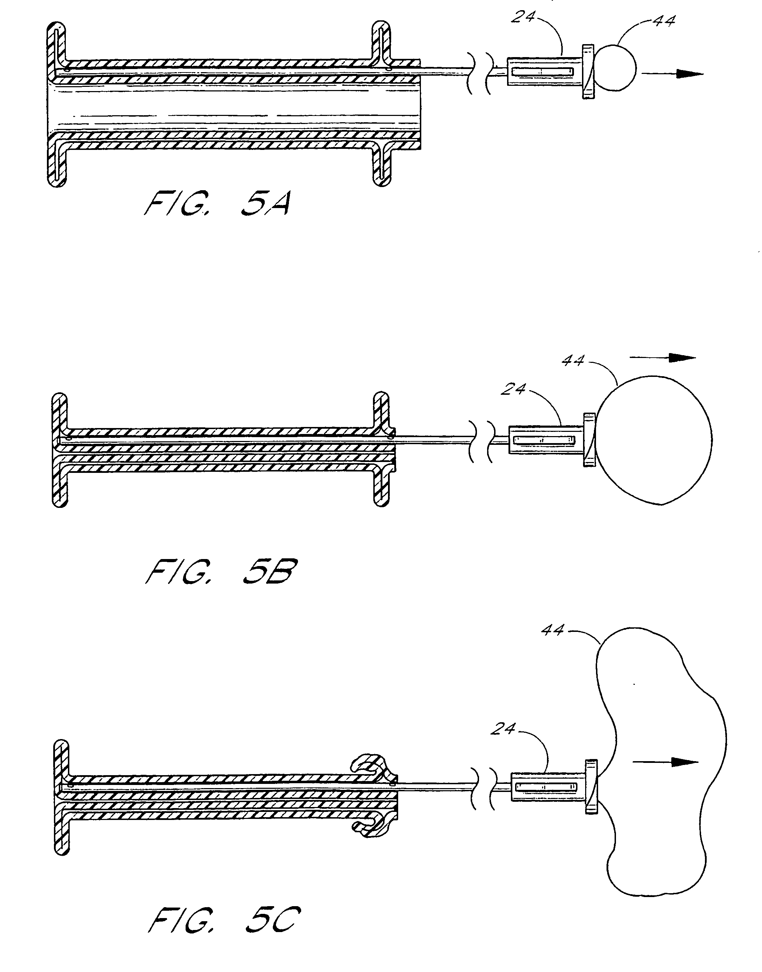

As seen in FIG. 2, the balloon 10 in a deflated state is placed around the tube 20 and inserted into a blood vessel 43, such as the aorta. When balloon 10 is inflated (Figure 3), as discussed above, the proximal portion 12 and the distal portion 14 occlude blood flow along the outside of the tubular member 16 while allowing blood to flow through inside the tubular member. As seen in FIGS. 1 and 3, the proximal portion 12 and the distal portion 14 are two partially occlusive portions that are spaced apart from one another. In the illustrated embodiment, the proximal portion 12 and the distal portion 14 are shaped like a donut. It will be recognized that other partially occlusive shapes are also contemplated. When the balloon 10 is inflated, the outer diameter of the proximal portion 12 and the distal portion 14 equals or exceeds the inner diameter of the blood vessel 34. It will be recognized that, depending on the size of the blood vessel contemplated, the proximal portion 12 and the distal portion 14 should be appropriately sized such that upon inflation, the proximal portion 12 and the distal portion 14 make respective seals 36, 36' between the outer edges of the proximal portion 12 and the distal portion 14 and the inner wall 30 of the blood vessel 34. As seen in FIGS. 1 and 3, when inflated, the proximal portion 12 and the distal portion 14 extend radially outwardly from the axis of the tubular member 12. The diameter of the tubular member 16 is smaller than the diameter of the proximal portion 12 and the distal portion 14, such that when the proximal portion 12 and the distal portion 14 come in contact with the inside wall 30 of the blood vessel 34, an area of hemostasis 32 is created between the outer wall of the tubular member 16 and the inner wall 30 of the blood vessel 34. The blood will continue to flow through the tubular member 16. However, the blood trapped in the area of hemostasis 32 will be static. Once the region of hemostasis is created, a bypass graft (not shown) can be attached to the blood vessel 34 (using a standard end-to-side anastomoses procedure) between the proximal and distal portions 12 and 14.

With reference to FIGS. 1, 4 and 5A-5C, the suture lumen 24 aspect will be described. In the illustrated embodiment of Figure 4, a plurality of holes 40 (Figure 4) are placed on a flange 42 at the proximal end of the balloon. Threaded through the holes 40 in a purse string arrangement and extending through the suture lumen 19 is a suture loop 44, a portion of which is accessible to the physician via a second lure connector 24. When a manual force is applied to the suture loop 44 after the device has been deflated, the loop 44 will cause the flange 42 and the proximal portion 12 to collapse to a configuration in which the balloon 10 can more easily be withdrawn through the incision. The suture loop 44 or a second suture loop may optionally be coupled to the distal portion 14, so that both portions 12, 14 can be caused to collapse.

Figures 5A-5C illustrate generally how the balloon 10 collapses as a force is applied to the suture loop 44 with the balloon in a deflated state. As best illustrated by Figure 5C, the balloon is preferably constructed such that the radially-extending walls of the proximal portion 12 fold over in the distal direction.

With reference to FIGS 6-8, a preferred method for manufacturing the balloon portion of the device will be described. A mandrel 50 may be used to manufacture the balloon member 52. The mandrel is preferably composed of stainless steel. During the manufacturing process, the mandrel 50 is appropriately dipped in a liquid polyethylene or other solution of low compliance biocompatible material a sufficient number of times to produce a wall thickness of approximately 0.4 mils to 0.7 mils (where 1 mil = 0.001 inches). The compliance is preferably selected such that the balloon stretches by approximately 10% to 20% when the balloon 10 is inflated. The use of a low-compliance material for this purpose reduces longitudinal stretching, and enables a small contact area to be maintained. The wall thicknesses are exaggerated throughout the drawings to facilitate visualization of the balloon's construction.

Following the dipping process, the balloon member 52 is a single, continuous one-piece member having an open end 54, a first elongated section 56, a second elongated section 60, and a rounded end portion 62. The balloon member 52 is subsequently removed from the mandrel 50. As seen in FIG. 7, the rounded end portion 62 is trimmed such that it is no longer enclosed but is open. As seen in FIG. 8, the open end 54 is then inverted inward, and the first elongated portion 56 is pulled through the center of the balloon member 52 such that the open end 54 aligns with the rounded end 62. In so doing, the first elongated section 56 forms the inner layer and the second elongated section 60 forms the outer layer of the balloon 10. Then the multi-lumen tube 20 is inserted between the layers of the first elongated section 56 and the second elongated section 60. The opening 26 and the opening 28 for inflation align with the proximal portion 12 and the distal portion 14, respectively. Thereafter, the edges of the open end 54 and the rounded end 62 are circumferentially sealed to one another using known sealing methods, such as RF welding, thermal bonding or adhesives.

It will be understood that an important use of the device 9 is for a cardiac bypass procedure. As seen in Figure 11, the balloon 10 is inserted into the descending aorta and is then inflated to create an area of hemostasis. In the illustrated embodiment, the balloon 10 is introduced into the aorta through a small incision 70 in the aortic wall using a direct access surgical procedure. As would be appreciated by a person skilled in the art, the balloon 10 could alternatively be introduced into the femoral artery via a percutaneous catheter and advanced to the aorta. In addition, a marker (not shown) placed on the tube 20 would indicate how far to advance the balloon 10, such that the balloon 10 is not inserted into the heart.

The length of the tubular member 16 is preferably sufficient to provide a working area to perform a quadruple bypass graft.

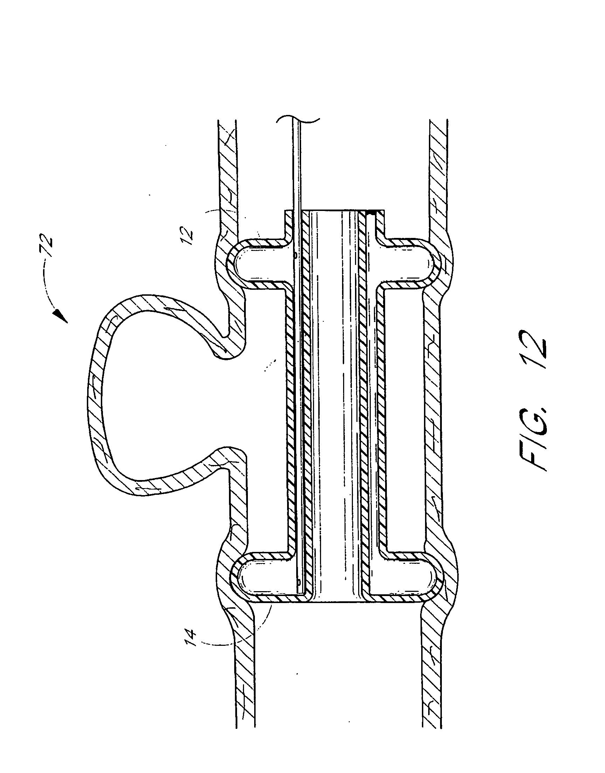

Another important use of the device 9 is to repair damages vessels. As illustrated in FIG. 12, the device 9 can be used to treat an aneurysm 72. To isolate the aneurysm 72, the balloon 10 is inserted (using either a direct access or a percutaneous procedure) such that the proximal and distal portions 12, 14 are positioned on opposite sides of the aneurysm, and the balloon 10 is then inflated. As will be appreciated by a person skilled in the art, the device 10 can also be used in any blood vessel where there is a need to isolate an area yet still allow passage of blood through the blood vessel.

Although a preferred embodiment of the invention has been described in detail, other implementations will be apparent to those skilled in the art. Accordingly, the scope of the present invention is intended to be defined only by reference to the appended claims.

## Claims

1. A partially occlusive blood flow-through device (9) for creating an area of hemostasis (32) within a blood vessel (34) without interrupting blood flow through the blood vessel (34), the device comprising:
- first and second partially occlusive portions (12, 14) that are spaced apart from one another, wherein the partially occlusive portions (12, 14) are inflatable to at least the inner diameter of the blood vessel (34); and
- a tubular member (16) that extends in a longitudinal direction and that interconnects the first and second partially occlusive portions (12, 14) and forms a conduit which allows the blood to flow through, wherein the diameter of the tubular member (16) is smaller than the diameter of the first and the second partially occlusive portions (12, 14); and
- wherein inflation of the first and second partially occlusive portions (12, 14) creates an area of hemostasis (32) between an outer surface of the tubular member (16) and the inner wall (30) of the blood vessel (34) while blood continues to flow through the tubular portion, **characterised in that** the first and second partially occlusive portions (12, 14) are made from a low compliance biocompatible material, the low compliance biocompatible material restricting stretching of the first and second partially occlusive portions (12, 14) in the longitudinal direction to enable a small contact area to be maintained between an inner wall (30) of the blood vessel and inflated first and second partially occlusive portions (12, 14), wherein said low compliance biocompatible material stretches by no more than about 10%-20% upon inflation.

2. The partially occlusive blood flow-through device of claim 1, wherein the tubular member (16) comprises inner and outer tubular portions (56, 60), one of the tubular portions within the other of the tubular portions, said inner tubular portion (56) forming a lumen for blood flow, said tubular portions (56, 60) being attached to each other.

3. The partially occlusive blood flow-through device of claim 2, wherein said occlusive portions (12, 14) and said tubular portions (56, 60) are of a single piece construction comprised of a single material.

4. The partially occlusive blood flow-through device of claims 1 to 3, wherein the partially occlusive portions (12, 14) are sized to create a region of hemostasis (32) within the human aorta.

5. The partially occlusive blood flow-through device of claims 1 to 4, wherein said device (9) further comprises a tube (20) that is coupled to the first and second partially occlusive portions (12, 14) and adapted to be inserted into an incision in the blood vessel (34) to deploy said partially occlusive portions (12, 14) within the blood vessel (34).

6. The partially occlusive blood flow-through device of daim 5, wherein said tube (20) has at least one inflation lumen formed therein, the inflation lumen fluidly coupled to the tubular member (16) and the first and second partially occlusive portions (12, 14).

7. The partially occlusive blood flow-through device of claim 6, wherein said tube (20) further comprises a lumen having a suture loop (44) therein, the suture loops (44) coupled to at least one of the inflatable partially occlusive portions (12, 14) such that the application of a force to the loop (44) from the outside the blood vessel (34) urges at the least one partially occlusive portion (12, 14) to a collapsed state.

8. The partially occlusive blood flow-through device according to any the of previous daims, wherein said device (9) further comprises means for collapsing the at least one of the partially occlusive portions (12, 14) to facilitate removal from the blood vessel (34).

## Patentansprüche

1. Eine partiell verschließende Blutdurchfluss-Vorrichtung (9) zur Schaffung eines Gebietes von Hämostase (32) innerhalb eines Blutgefäßes (34), ohne den Blutfluss durch das Blutgefäß (34) zu unterbrechen, wobei die Vorrichtung umfasst:
- erste und zweite partiell verschließende Teile (12, 14) die von einander getrennt angeordnet sind, wobei die partiell verschließenden Teile (12, 14) auf mindestens den Innendurchmesser des Blutgefäßes (34) aufblasbar sind; und
- ein röhrenförmiges Glied (16), das sich in Längsrichtung erstreckt und die ersten und zweiten partiell verschließenden Teile (12, 14) miteinander verbindet und eine Leitung bildet, die den Durchfluss des Blutes erlaubt, wobei der Durchmesser des röhrenförmigen Gliedes (16) kleiner ist als der Durchmesser des ersten und zweiten partiell verschließenden Teiles (12, 14); und
- wobei das Aufblasen des ersten und zweiten partiell verschließenden Teiles (12, 14) ein Gebiet der Hämostase (32) zwischen einer äußeren Oberfläche des röhrenförmigen Glieds (16) und der inneren Wand (30) des Blutgefäßes (34) erzeugt, während das Blut weiterhin durch das röhrenförmige Teil fließt,
**dadurch gekennzeichnet, dass** die ersten und zweiten partiell verschließenden Teile (12, 14) aus einem biokompatiblen Material mit geringer elastischer Verformbarkeit sind, wobei das biokompatible Material mit der geringen Verformbarkeit das Dehnen der ersten und zweiten partiell verschließenden Teile (12, 14) in Längsrichtung einschränkt, so dass eine kleine Kontaktzone zwischen einer Innenwand (30) des Blutgefäßes und den aufgeblasenen ersten und zweiten partiell verschließenden Teilen (12, 14) aufrechterhalten bleibt, wobei das biokompatible Material mit der geringen Verformbarkeit sich nach dem Aufblasen um nicht mehr als 10 bis 20 % dehnt.

2. Die partiell verschließende Blutdurchfluss-Vorrichtung nach Anspruch 1, wobei das röhrenförmige Glied (16) innere und äußere röhrenförmige Teile (56, 60) umfasst, wobei eines der röhrenförmigen Teile in dem anderen röhrenförmigen Teil angebracht ist und das innere röhrenförmige Teil (56) ein Lumen für den Blutfluss bildet, und wobei die röhrenförmigen Teile (56, 60) aneinander geheftet sind.

3. Die partiell verschließende Blutdurchfluss-Vorrichtung nach Anspruch 2, wobei die verschließenden Teile (12, 14) und die röhrenförmigen Teile (56, 60) aus einem einzigen Element eines einzigen Materials bestehen.

4. Die partiell verschließende Blutdurchfluss-Vorrichtung nach Anspruch 1 bis 3, wobei die partiell verschließenden Teile (12, 14) so bemessen sind, dass sie innerhalb der menschlichen Aorta einen Bereich der Hämostase (32) erzeugen.

5. Die partiell verschließende Blutdurchfluss-Vorrichtung nach Anspruch 1 bis 4, wobei die Vorrichtung (9) weiterhin eine Röhre (20) umfasst, die an die ersten und zweiten partiell verschließenden Teile (12, 14) angebracht ist und so ausgestaltet ist, dass sie in einen Einschnitt in das Blutgefäß (34) eingeführt werden kann, so dass die partiell verschließenden Teile (12, 14) innerhalb des Blutgefäßes (34) auseinander gezogen werden.

6. Die partiell verschließende Blutdurchfluss-Vorrichtung nach Anspruch 5, wobei die Röhre (20) zumindest ein darin gebildetes Aufblaslumen hat, und wobei das Aufblaslumen fluidal an das röhrenförmige Glied (16) und die ersten und zweiten partiell verschließenden Teile (12, 14) angebracht ist.

7. Die partiell verschließende Blutdurchfluss-Vorrichtung nach Anspruch 6, wobei die Röhre (20) weiterhin ein Lumen mit einer Schlaufennaht (44) darin aufweist, wobei die Schlaufennaht (44) mit mindestens einem der aufblasbaren partiell verschließenden Teile (12, 14) so verbunden ist, dass die Anwendung einer Kraft auf die Schlaufe (44) von außerhalb des Blutgefäßes (34) zumindestens ein partiell verschließendes Teil (12, 14) in einen kollabierten Zustand zwingt.

8. Die partiell verschließende Blutdurchfluss-Vorrichtung nach einem der vorhergehenden Ansprüche, wobei die Vorrichtung (9) außerdem Mittel umfasst, um mindesten eines der partiell verschließenden Teile (12, 14) zum Kollabieren zu bringen, um **dadurch** die Entfernung aus dem Blutgefäß (34) zu erleichtem.

## Revendications

1. Dispositif d'écoulement sanguin partiellement occlusif (9) destiné à créer une zone d'hémostase (32) à l'intérieur d'un vaisseau sanguin (34) sans interrompre le flux sanguin à travers le vaisseau sanguin (34), le dispositif comprenant
- une première et une seconde partie partiellement occlusive (12, 14) qui sont disposées à distance l'une de l'autre, dans lequel les parties partiellement occlusives (12, 14) sont gonflables jusqu'à au moins le diamètre interne du vaisseau sanguin (34) ; et
- un élément tubulaire (16) qui s'étend dans une direction longitudinale et qui relie entre elles les première et seconde parties partiellement occlusives (12, 14) et forme un conduit qui permet au sang de s'écouler, dans lequel le diamètre de l'élément tubulaire (16) est inférieur au diamètre des première et seconde parties partiellement occlusives (12, 14) ; et
- dans lequel le gonflage des première et seconde parties partiellement occlusives (12, 14) crée une zone d'hémostase (32) entre une surface externe de l'élément tubulaire (16) et la paroi interne (30) du vaisseau sanguin (34) tandis que le sang continue de s'écouler à travers la partie tubulaire, **caractérisé en ce que** les première et seconde parties partiellement occlusives (12, 14) sont fabriquées dans un matériau biocompatible à faible élasticité, le matériau biocompatible à faible élasticité limitant l'étirement des première et seconde parties partiellement occlusives (12, 14) dans la direction longitudinale pour permettre de conserver une petite zone de contact entre une paroi interne (30) du vaisseau sanguin et les première et seconde parties partiellement occlusives (12, 14) gonflées, dans lequel ledit matériau biocompatible à faible élasticité s'étire au maximum d'environ 10 à 20 % lors du gonflage.

2. Dispositif d'écoulement sanguin partiellement occlusif selon la revendication 1, dans lequel l'élément tubulaire (16) comprend des parties tubulaires interne et externe (56, 60), l'une des parties tubulaires se trouvant à l'intérieur de l'autre des parties tubulaires, ladite partie tubulaire interne (56) formant une lumière pour le flux sanguin, lesdites parties tubulaires (56, 60) étant fixées l'une à l'autre.

3. Dispositif d'écoulement sanguin partiellement occlusif selon la revendication 2, dans lequel lesdites parties occlusives (12, 14) et lesdites parties tubulaires (56, 60) forment une construction monobloc composée d'un seul matériau.

4. Dispositif d'écoulement sanguin partiellement occlusif selon les revendications 1 à 3, dans lequel les parties partiellement occlusives (12, 14) sont dimensionnées de façon à créer une région d'hémostase (32) à l'intérieur de l'aorte de l'être humain.

5. Dispositif d'écoulement sanguin partiellement occlusif selon les revendications 1 à 4, dans lequel ledit dispositif (9) comprend en outre un tube (20) qui est couplé aux première et seconde parties partiellement occlusives (12, 14) et adapté pour être inséré dans une incision effectuée dans le vaisseau sanguin (34) afin de déployer lesdites parties partiellement occlusives (12, 14) à l'intérieur du vaisseau sanguin (34).

6. Dispositif d'écoulement sanguin partiellement occlusif selon la revendication 5, dans lequel ledit tube (20) présente au moins une lumière de gonflage formée à l'intérieur de celui-ci, la lumière de gonflage étant couplée de manière fluidique à l'élément tubulaire (16) et aux première et seconde parties partiellement occlusives (12, 14).

7. Dispositif d'écoulement sanguin partiellement occlusif selon la revendication 6, dans lequel ledit tube (20) comprend en outre une lumière présentant une boucle de suture (44) à l'intérieur de celle-ci, les boucles de suture (44) étant couplées à au moins l'une des parties partiellement occlusives (12, 14) gonflables, de sorte que l'application d'une force sur la boucle (44) depuis l'extérieur du vaisseau sanguin (34) pousse au moins une partie partiellement occlusive (12, 14) dans un état affaissé.

8. Dispositif d'écoulement sanguin partiellement occlusif selon l'une quelconque des revendications précédentes, dans lequel ledit dispositif (9) comprend en outre des moyens destinés à affaisser la ou chacune des parties partiellement occlusives (12, 14) pour faciliter son retrait du vaisseau sanguin (34).
